# FASCICULE DE BREVET EUROPEEN

(11) **EP 0 659 087 B1**
(45) Date de publication et mention de la délivrance du brevet: **04.06.1997**
(21) Numéro de dépôt: 93918809.0
(22) Date de dépôt: 07.09.1993
(51) Int. Cl.: A61L 15/07, C08G 18/42, C08L 75/06

(54) **COMBINAISONS HOMOGENES DE MATIERES, SOUPLES OU RIGIDES, MOULABLES ET ADHESIVES SOUS L'EFFET D'UNE TEMPERATURE INFERIEURE A 90o**
HOMOGENE ZUSAMMENSETZUNGEN WEICHER ODER HARTER STOFFE DIE BEI TEMPERATUREN UNTER 90 oC KLEBRIG UND VERFOMBAR SIND
HOMOGENEOUS COMBINATIONS OF FLEXIBLE OR RIGID MATERIALS MOULDABLE AND ADHESIVE AT TEMPERATURES BELOW 90 oC

(30) Priorité: 07.09.1992 BE 9200786
(43) Date de publication de la demande: 28.06.1995
(73) Titulaire: L'UNIVERSITE DE LIEGE, B-4000 Liège (BE); Liegeois, Jean-Marie, B-4654 Charneux-Herve (BE)
(72) Inventeur: LIEGEOIS, Jean-Marie, B-4654 Charneux/Herve (BE)
(74) Mandataire: Van Malderen, Joelle
(86) Numéro de dépôt international: BE9300055
(87) Numéro de publication internationale: WO9405338

(56) Documents cités:
- EP-A- 0 086 686
- EP-A- 0 169 037
- EP-A- 0 235 500
- EP-A- 0 443 757
- WO-A-91/09909
- GB-A- 2 021 600
- US-A- 4 326 509

## Description

La présente invention se rapporte à de nouveaux alliages sous différentes formes de réseaux polymères interpénétrés (IPN) principalement sous forme semi-IPN ou IPN thermoplastique, en feuilles, plaques ou masses thermomalléables et thermoadhésives, souples ou rigides et à prise rapide ainsi qu'à des procédés de préparation de ces alliages et de leur application éventuelle sur un substrat textile.

A côté des applications usuelles des matières plastiques, il y a divers domaines d'application potentielle où il est demandé de réaliser chaque fois une exécution unique d'un objet ou d'un assemblage dans une matière que l'on désire pouvoir mouler, former, modeler, de préférence à la main et par un traitement préalable simple tel qu'un réchauffage à une température facilement accessible. Il peut s'agir aussi d'une matière que l'on désire appliquer entre deux objets qui se rapportent mal pour les unir ensemble et où notamment, des adhésifs usuels ne conviennent pas parce qu'il y des cavités à combler par exemple.

Par traitement préparatoire à leur utilisation, ces matières doivent donc acquérir une malléabilité adéquate ainsi qu'un pouvoir adhésif sur elles-mêmes et éventuellement sur d'autres corps.Le traitement préparatoire envisagé ici se résume à une mise en température qui sera précisée plus loin, à l'exclusion d'usage de tout solvant ou adhésif externe.

L'application visée peut porter sur un objet ou une réalisation aussi rigide que possible tout comme on peut rechercher dans l'application, une certaine souplesse du matériau à pouvoir choisir suivant les cas.

De telles applications sont souvent recherchées par des personnes n'ayant pas nécessairement une habileté manuelle poussée, qui veulent exercer elles-mêmes sans moyens techniques conséquents et en réalisant le moulage, le modelage ou l'assemblage principalement à la main avec l'aide éventuelle de quelques instruments simples tels que ciseaux, pinces et supports. Sans doute, les applications visées ici peuvent être pratiquées par des professionnels également exerçant dans un environnement industriel mais ce n'est pas requis.

Il résulte que ces applications de formage, moulage, collage doivent pouvoir être réalisées à partir d'une température de traitement préparatoire qui reste accessible sans risque de brûlures ou d'autre inconvénient pouvant causer une maladresse ou un accident.

La matière employée doit perdre son caractère adhésif une fois revenue à température ambiante et conserver aussi la forme qui lui a été donnée de même qu'elle doit être apte à résister aux contraintes mécaniques auxquelles elle pourrait être exposée et ne pas perdre sa forme jusqu'à une température dictée par l'application.

Un domaine de température pour le traitement préparatoire allant de 40 à 80°C parait convenable et en tout cas ne dépassant pas 100°C de sorte que cette préparation peut se faire aisément et relativement rapidement en utilisant de l'eau chaude par exemple. Il va de soi que des sources de chaleur alternatives peuvent être utilisées également comme l'étuve, le four, le pistolet chauffant ou le champ de micro-ondes.

Vu les applications visées, il est aussi recommandé que ces matières soient inertes et ne relarguent pas de substances toxiques, irritantes ou d'un autre caractère nocif de sorte que l'applicateur puisse les utiliser sans moyen de protection particulier.

Ces produits doivent aussi être stables pour une longue durée sans être sujet à un vieillissement chimique ni de préférence également à tout vieillissement physique.

Il ne faut pas non plus devoir recourir pour ce faire à un mode d'emballage sophistiqué tel que des pochettes hermétiques qui risquent de se déchirer ou qui compromettent l'usage de la matière une fois la pochette ouverte.

Il existe déjà quelques domaines d'application du type de produits visés par la présente invention où on utilise des matières formables et thermoadhésives ou non à partir d'une température de traitement allant de 70 à 80°C environ et qui sont essentiellement confinés dans des usages où on recherche une grande rigidité. Il s'agit principalement de la physiothérapie où l'on réalise ainsi déjà des attelles, orthèses et autres moyens de support rigides, de l'orthopédie intéressée par des bandages de contention cylindrique dits thermoplastiques ou par des prothèses externes du même matériau, mais aussi du domaine de la décoration, où des décors de théâtre, des masques et objets divers essentiellement en relief sont ainsi façonnés.

Pour obtenir l'effet de formabilité seulement, par traitement à 70-75°C environ, on connaît actuellement le trans 1-4 polyisoprène, mieux connu sous le nom de marque ORTHOPLAST notamment, et qui permet de réaliser des attelles et des orthèses avec une grande facilité du fait de la bonne malléabilité du trans 1-4 polyisoprène à l'état fondu. Toutefois, le trans 1-4 polyisoprène ne présente pas d'adhésivité sur lui-même ni sur un corps étranger et il faut utiliser du chloroforme pour souder deux éléments ensemble.

Pour obtenir l'effet de formabilité et de thermoadhésivité réunis dans le domaine de température souhaité, on dispose également de polymères linéaires d'ester cyclique caractérisés par les fonctions "COO" séparées par des radicaux méthylènes comprenant de 2 à 7 carbones dont le principal représentant disponible est le polymère du 2-oxepanone mieux connu sous l'appellation polycaprolactone (PCL). Le brevet des Etats-Unis d'Amérique 3,692,023 suggère l'emploi de ce polymère pour l'obtention de matériel de contention. Par ailleurs, le brevet des Etats-Unis d'Amérique 4,273,115 préconise un tricot Raschel particulier partiellement imprégné de ce polymère PCL de poids moléculaire 40 000 environ comme matériel de contention cylindrique. Enfin, dans le brevet des Etats-Unis d'Amérique 4,316,457 on a proposé la préparation d'un polyuréthanne thermoplastique linéaire d'un poids moléculaire de 40000 à 45 000, synthétisé en deux étapes où un prépolymère à deux moles d'un diisocyanate et une mole d'un polyester diol est additionné d'une mole du polyester diol avant imprégnation d'un tricot Raschel pour donner l'extension de chaîne en achevant la polymérisation sur celui-ci en même temps que le solvant s'évapore.

Tels que décrits, ces polyesters deviennent fluides à la température de fusion et ont une adhésivité excessive sur tout objet auquel il viennent en contact à l'état fondu. Cette adhésivité à l'état fondu qui entraîne un dépôt de matière est néanmoins perdue après durcissement par refroidissement. C'est pourquoi, l'utilisation de ces polyesters a nécessité diverses adaptations qui se résument en trois catégories.

La première catégorie porte sur le recours à des moyens externes. Dans le brevet des États-Unis d'Amérique 3,692,023, on utilise une couche de base souple pour éviter que le polymère adhère à la peau, aux poils et aux cheveux qu'il recouvre lorsqu'on l'y applique. Dans le même brevet, on envisage de presser le même polymère en feuille, sur un support ou en sandwich fait d'un tissu, tricoté, tissé ou non tissé dont le rôle est d'empêcher l'écoulement de la matière à l'état fondu. Dans les brevets des Etats-Unis d'Amérique 4,273,115 et 4,316,457 relatifs aux bandages orthopédiques, on utilise une pellicule de séparation en polyéthylène qui est bobinée en même temps que le bandage pour éviter la prise en masse de celui-ci au moment de son application. Le brevet des Etats-Unis d'Amérique 4,143,655 préconise un autre type de séparateur à passages multiples tandis que le brevet des Etats-Unis d'Amérique 4,454,873 permet de supprimer l'effet gênant d'un séparateur par application d'un film polymère antiadhérant et hydrosoluble sur le matériau du bandage lui-même.Cette dernière solution a cependant l'inconvénient de contaminer progressivement l'eau du bain-marie de préparation et de réduire considérablement la résistance interlaminaire entre les différents plis d'une contention cylindrique notamment.

La seconde catégorie porte sur des adaptations physiques internes au matériau qui contient ces polyesters.Ainsi des teneurs très élevées jusqu'à 50 pour cent de charges minérales, de préférence finement divisées, permettent d'augmenter la viscosité de ces polyesters à l'état fondu sans toutefois empêcher une plaque de 3mm par exemple de s'allonger sous son propre poids.Cette même plaque, à l'état fondu, adhère sur elle-même d'une manière qui ne permet pas de corriger une erreur de positionnement. Le brevet européen EP-A-0 169 037 utilise ce principe de sorte que la pégosité est réduite par le choix des fillers en haute concentration tandis que la déformation à l'état fondu (stretch) est contrôlée par la réticulation avec un caoutchouc thermoplastique prémélangé suivant la troisième catégorie d'adaptations décrite ci-après.On a pu remarquer aussi que le type de tricot Raschel à brins volumineux faits de fibres coupées décrit dans les brevets des Etats-Unis d'Amérique 4,273,115 et 4,316,457 est absolument nécessaire pour supprimer l'effet de la trop grande fluidité des polycaprolactones et des polyuréthannes linéaires s'y rapportant, appliqués dans un bandage orthopédique par exemple. Ces brins très ouverts ont une densité apparente très faible et offrent un volume entre fibres d'environ 80 pour cent. Le brevet des Etats-Unis d'Amérique 4,273,115 précise d'ailleurs que les brins de fibres ne peuvent être que partiellement imprégnés du polymère.La résistance mécanique de ces brins, nécessairement peu noués et partiellement imprégnés, est dès lors assez faible, ce qui est constaté en particulier sur le produit fini imprégné de résine et que l'on peut facilement déchirer à la main.Les éléments de brin étant ainsi enrobés de polymère, l'ensemble s'oppose à l'écoulement fluide du composant thermoadhésif. Celui-ci reste néanmoins très déformable d'une manière plastique, et l'usage d'une bobine de ce matériau traité à température, conduit rapidement à une masse compacte qu'il devient très difficile de dérouler si l'on n'a pas au préalable, posé un film intercalaire de polyéthylène qui est bobiné en même temps que le bandage proprement dit.

Enfin, la troisième catégorie d'adaptations vise à conférer à la polycaprolactone une résistance à la déformation à la température d'utilisation, voire un effet de mémoire élastique par divers moyens de réticulations physiques ou chimiques ou par un mélange mécanique avec un autre polymère suivi d'une réticulation physique ou chimique. Ces techniques d'adaptation de la polycaprolactone sont décrites dans les brevets des Etats-Unis d'Amérique 4,240,415 de L.H. Wartman (réticulation chimique par électrons), dans le brevet des Etats-Unis d'Amérique 4,175,177 de J.E. Potts (modification de la polycaprolactone sous forme de copolymère réticulable), dans le brevet des Etats-Unis d'Amérique 4,483,333 de L.H. Wartman (mélange et réticulation physique par le polyéthylène), dans le brevet européen 0 235 500 de R.L. Mahlon (mélange mécanique avec réticulation physique par les segments durs), dans le brevet français 2,519,992 de H.Grouiller (mélange et réticulation chimique du polyuréthanne), dans le brevet européen EP-A-0 086 686 de H.Grouiller (mélange mécanique avec un élastomère miscible), dans le brevet WO-A-9 109 909 de Polysar (mélange mécanique avec réticulation physique par le terpolymère de haut Tg), dans le brevet des Etats-Unis d'Amérique 4,326,509 d'Usukura (mélange mécanique et réticulation physique par la cristallinité de la résine ajoutée au polyester linéaire saturé).

Les objectifs actuellement visés dans les applications basées sur ce savoir-faire ne sont pas satisfaits par ces polyesters aliphatiques ou ces polyuréthannes linéaires qui s'y rapportent ni par les simples mélanges de polymères décrits plus haut et surtout si l'application concerne le bandage orthopédique eu égard notamment aux quantités excessives de solvants qui seraient nécessaires. En effet, lorsque la PCL n'est pas réticulée par les techniques ci-dessus, sa fluidité à chaud fait qu'une plaque se déforme sous son propre poids et son adhésivité entraîne un transfert de matières sur les mains de l'utilisateur et sur ses outils sans toutefois donner lieu à une adhésion permanente.En outre, ils redurcissent, pendant le refroidissement, à une vitesse qui ne permet pas toujours à l'opérateur d'achever son travail sans réchauffer à nouveau localement ou totalement.

Si on essaie d'appliquer les polymères décrits dans les brevets des Etats-Unis d'Amérique 4,273,115 et 4,316,457 sur un tissu ou un tricot à mailles ouvertes notamment, et de haute ténacité ce qui n'est possible qu'en choisissant des brins plus serrés, mieux noués et faits de fibres continues, on est obligé, pour obtenir le même poids spécifique de résine thermoadhésive, d'enrober davantage l'extérieur du brin duquel, la résine qui n'est plus retenue par les fibres peut se déplacer de manière indésirable et être transférée sur les outils ou les mains de l'opérateur pendant l'application.

En outre, les tricots à brins volumineux tels que recommandés dans ces brevets sont d'une souplesse qui ne permet pas de les manipuler en largeur inférieure à un mètre environ. Dans ces conditions déjà, il y a rapprochement des brins de chaîne sous l'effet de la traction et du poids lorsqu'on imprègne par les procédés verticaux usuels également décrits dans ces mêmes brevets.Des bandages de largeurs usuelles sont donc obtenus dans ce cas, par découpe longitudinale après imprégnation du textile en grande largeur et séchage. Cette découpe résulte sur des aspérités à l'endroit des brins transversaux qui ont été tranchés. Il y a un intérêt évident d'obtenir un bandage en largeur utile et bordé de lisières uniformes exemptes d'aspérités. Toutefois, dans la configuration textile nécessaire aux polymères des brevets cités les bandes de petites largeurs ne sont pas assez soutenues et le produit fini se retrouve sensiblement plus étroit qu'au point de départ. On observe d'ailleurs parallèlement, avec ce type de tricot volumineux fait de brins à fibres coupées, un allongement dans l'autre direction qui est rendu au moment du chauffage préparatoire à l'utilisation. Les substrats textiles nécessaires pour les polymères du savoir-faire antérieur ne permettent donc pas d'obtenir un produit stable dimensionnellement, il est nécessaire de préparer les bandes par découpe après dépot du polymère. Si par ailleurs, on est intéressé par ces tricots imprégnés non pas dans l'optique d'un bandage circulaire mais bien de feuilles pour attelles et orthèses, l'opérateur doit tenir compte du retrait atteignant parfois 10 pour cent, qui se produit lors du chauffage préparatoire de la feuille, ce qui est gênant. Il y a là aussi intérêt à pouvoir utiliser des substrats textiles moins déformables.

Les polymères linéaires du savoir-faire antérieur sont aussi caractérisés par des propriétés thermiques comme la température de ramollissement, la température de fusion ou la vitesse de redurcissement, bien fixées. Par la spécificité des nouveaux besoins, il est devenu nécessaire de pouvoir agir sur ces caractéristiques et de préférence, indépendamment de toute autre action sur la rigidité ou la résistance mécanique.

Enfin, il a pu être constaté que les polymères linéaires notamment du savoir-faire antérieur subissent un vieillissement physique au point qu'au terme d'une année environ il devient parfois possible de casser à la main une plaque de 3 mm, celle-ci résistant à l'effort humain lorsqu'elle est fraîchement moulée.

Dès lors que les applications des produits visés par la présente invention comprennent un cycle de chauffage et de refroidissement, il est utile de définir au moins quatre températures caractéristiques dont il est intéressant d'examiner aussi les valeurs pour apprécier les avantages procurés par l'invention au-delà du simple contrôle de la fluidité et de la thermoadhésivité.

La température (T1) de ramollissement est la température à laquelle le matériau commence à perdre sa rigidité. Elle peut être déterminée notamment par méthode ASTM 1043-87 ou 1053-89 en prenant le point d'inflexion de la courbe de variation du module avec la température ou la température à laquelle le module d'élasticité a chuté de moitié par rapport à sa valeur à 22°C.

La température (T2) de traitement pour l'application est la température convenable à laquelle il faut porter le matériau pour que celui-ci acquière en un temps raisonnable les caractéristiques de formabilité et d'adhésivité recherchées et les conserve suffisamment pendant le temps nécessaire à l'achèvement de l'application. Il est à noter que pendant l'application, la matière refroidit de manière naturelle ou forcée. Suivant l'épaisseur de la matière et la température d'ambiance, cette température T2 sera plus ou moins élevée, nécessairement au-delà de la température thermodynamique de fusion du constituant semi-cristallin dans la combinaison.

La température (T3) est la température à laquelle, lors du refroidissement de la matière, normalement après la fin de l'application, la matière commence à perdre son pouvoir adhésif.

La température (T4) est la température à laquelle lors du refroidisssement de la matière, normalement après la fin de l'application, la matière commence à perdre son aptitude à être formée par accroissement de sa rigidité.

Dans la pratique, on fait plus souvent référence au temps (t3) après lequel la matière n'est plus adhésive et au temps (t4) après lequel la matière n'est plus déformable lorsqu'elle refroidit en épaisseur donnée, dans une ambiance donnée et à partir d'une température de traitement donnée. Dans ces conditions, il y a correspondance entre t3 et T3 ainsi qu'entre t4 et T4.

Cette distinction des quatre températures ci-dessus permet de préciser les caractéristiques optimales des produits visés, de ce point de vue.

D'une manière générale, il faut que la température T1 soit la plus élevée possible au-delà de la température ambiante, que la température T2 soit la moins élevée possible pour permettre une application aisée sans risque de brûlures, ce qui est particulièrement important dans le cas du bandage orthopédique.Il faut que la température T3 soit la plus basse possible pour donner un délai maximum t3 à l'opérateur chargé de l'application. La valeur optimale de T4 est fonction de l'application et du temps t4 que l'opérateur souhaite se réserver pour achever son travail. Le temps t4 doit être suffisant mais pas au point que l'opérateur doive attendre pour juger de son résultat.

Comme déjà dit, les polymères linéaires d'estercyclique du savoir-faire antérieur offrent peu de possibilité de changement de ces températures caractéristiques qui sont assez élevées eu égard aux conditions d'application.Ainsi des températures de traitement de 68 à 74°C sont recommandées dans la pratique ce qui crée un problème pendant le premier moment de l'utilisation du matériau. Les températures T3 et T4 sont élevées également de sorte que les temps de travail t3 et t4 sont souvent trop courts et ceci d'autant plus qu'un laps de temps plus long est sacrifié avant de pouvoir manipuler le matériau qui est trop chaud.On arrive, dans la pratique à compenser cet inconvénient par un retraitement général ou localisé à la température T2 choisie éventuellement plus élevée encore, en cours de travail, mais cela entraîne des imperfections, imprécisions et parfois un état de nervosité des opérateurs peu expérimentés.

En résumé, il y a pour chaque type d'application, une valeur optimale de la température T4 ou du temps t4 qui dépend notamment de la dimension et de la complexité du travail à réaliser. T1 doit être aussi élevé que possible et T2, supérieur à T1, le moins élevé possible, de sorte qu'une mesure de l'efficacité d'un matériau peut être traduite dans l'écart entre T1 et T2 qui doit être le plus faible possible. Dans le savoir-faire antérieur, T3 ne peut être que supérieur à T4 puisqu'il y a toujours un gradient de température entre le coeur du matériau et sa surface. Il y a intérêt à rapprocher T3 de T4 ou t3 de t4, et même d'avoir parfois inversion de l'ordre naturel de ces temps et températures de sorte que l'adhérence est possible jusqu'au dernier moment de la formabilité.

Il est à présent clair que les caractéristiques d'adhésivité et de fluidité du matériau introduites plus haut sont à prendre en considération, respectivement dans les intervalles de temps t3 et t4.

L'objet de la présente invention est de procurer des matières devenant facilement malléables ou moulables à la main jusqu'à de grandes dimensions de un ou deux mètres carrés, à partir d'un traitement à une température la plus basse possible en dessous de 100°C, tout en gardant les propriétés de température ambiante jusqu'à une température la plus elevée possible au-dessus de la température ambiante.

Un autre objet de la présente invention est de procurer ces matières sous forme de bandages orthopédiques thermoplastiques, légers et aérés, présentant sans devoir nécessairement recourir à une pellicule d'un film intercalaire, une bonne résistance interlaminaire, une bonne rigidité et une bonne résistance à la déchirure, sans que la bande ne devienne une masse compacte ni transfère la résine qu'elle contient sur des corps autres qu'elle-même pendant son application à chaud.

Un autre objet de la présente invention est de procurer également ces matières sous forme de bandages souples ou semi-rigides qui peuvent être laminés par traitement à température et qui redeviennent secs à froids.

Un autre objet de la présente invention est de procurer ces matières sous forme de plaques recyclables en 3mm d'épaisseur environ, garnies de passages ouverts ou non qui par traitement à une température inférieure à 100°C se prêtent de manière stable au formage et à la déformation, sans marquage prononcé par les doigts, et adhérent sur elles-mêmes d'une manière à pouvoir être désolidarisées si nécessaire, sans laisser de traces importantes, et qui ont une rigidité caractérisée par un module de flexion d'au moins 500 MPa.

Un autre objet de la présente invention est de procurer ce même type de plaque de rigidité ou souplesse variable caractérisées par un module d'élasticité en flexion pouvant aller de 50 à 600 MPa en l'absence de charges.

Un autre objet de la présente invention est de procurer ces matières sous forme de plaques et feuilles composites ou non facilement moulables et adhérant sur elles-mêmes et qui gardent leur forme pendant le moulage sans risque de déformation incontrôlable due soit à une trop grande fluidité, soit à un effet de mémoire élastique, de sorte qu'un seul opérateur peut préparer par laminage, une pièce de grande dimension comme un lombostat, un décor de théâtre ou l'empreinte d'un objet de plus d'un mètre de dimension moyenne.

Un autre objet de la présente invention est de procurer ces matières sous forme de masses thermoadhésives, souples ou rigides et à prise rapide, pouvant unir des objets rugueux ou qui ne se rapportent pas, en y adhérant à une température qui permet de les manipuler facilement.

Un autre objet de la présente invention est de pouvoir obtenir ces matières thermoadhésives telles qu'elles puissent être adhésives aussi longtemps qu'elles sont malléables.

Un autre objet de la présente invention est d'obtenir ces matières par des procédés économiques faisant intervenir le moins possible ou pas de solvant.

Un autre objet de la présente invention est de rendre ces matières recyclables et réutilisables.

Dans la présente invention, on a obtenu plusieurs manières de contrôler la fluidité, l'adhésivité, la rigidité et les températures caractéristiques de matériaux themoadhésifs sous forme de masse granulée ou non, de plaques, de feuilles ou encore de textiles à mailles ouvertes ou non enduits de ces matières, formables et moulables dans les conditions décrites plus haut d'un travail aisé à une température qui ne gêne pas une application manuelle notamment.

Selon la présente invention, on a trouvé de manière inattendue que certaines combinaisons de type IPN, c'est à dire, où notamment l'un des constituants au moins est synthétisé en présence de l'autre, et associant une structure de polymère amorphe ou semi-cristallin qui a dans la combinaison un caractère élastovisqueux à caoutchoutique au-dessus d'une certaine température toutefois inférieure à 80°C, avec une proportion variable suivant les cas, de structures polymères thermoplastiques essentiellement semi-cristallines et contenant une quantité minimum d'unités structurales de type ester aliphatique, elle-mêmes relativement fluides à l'état fondu lorsqu'elles n'entrent pas dans la combinaison, permettent de pallier les difficultés citées plus haut d'une trop grande fluidité et d'une adhésivité excessive, tout en donnant des possibilités nouvelles de moduler ces caractéristiques ainsi que d'autres caractéristiques utiles dans l'application ou l'usage des matières qui en découlent comme la rigidité et les températures T1,T2,T3 et T4.

Dans la suite de cette description, on appellera respectivement, "premier constituant" et "second constituant", les deux constituants de la combinaison définis plus haut.

La combinaison de la présente invention comprend un premier constituant polymérique amorphe ou semi-cristallin ayant dépassé son point de ramollissement thermique au moins à la température T2 et présentant à cette même température au moins, un comportement élastovisqueux à caoutchoutique, et un second constituant essentiellement semi-cristallin d'un contenu en unités structurales de type ester aliphatique d'au moins 80 pour cent environ et présentant à la température T2, un comportement essentiellement plastique et telle que l'un au moins des deux constituants ayant été synthétisé en présence de l'autre, on aboutisse à une morphologie de type semi-IPN, IPN thermoplastique ou homo-IPN.

On a trouvé en effet que ces combinaisons très distinctes des simples mélanges mécaniques de polymères confèrent des propriétés nouvelles souvent inaccessibles par ces mélanges. Conformément à la topologie définissant les IPN, on a observé également que ces nouvelles combinaisons présentent une morphologie typique manifestée notamment par microscopie électronique et par analyse mécanique dynamique et qui ne s'apparente pas à celle des mélanges décrits dans le savoir-faire antérieur.

Les combinaisons telles que décrites ci-avant se sont avérées thermoadhésives de manière tout à fait satisfaisante sans présenter l'inconvénient d'une déformation visqueuse incontrôlable. Par le choix de la proportion et des éléments du premier constituant, et aussi dans une certaine mesure par le choix des éléments du second constituant, on a remarqué qu'il était aussi possible d'adapter les températures caractéristiques, la souplesse pendant l'application et la rigidité du produit obtenu, selon les besoins.

Selon la présente invention, le second constituant est choisi d'une manière qu'il ait des propriétés thermoadhésives intrinsèques alors que le premier constituant n'en a pas nécessairement. Inversément on a trouvé que le premier constituant doit conférer à la combinaison un caractère élastovisqueux lorsque celle-ci est soumise à la température de traitement préparatoire, ce qui a été obtenu avec certains premiers constituants présentant eux-mêmes un caractère élastovisqueux à caoutchoutique, alors que le second constituant ne peut être que plastique à cette même température.

Ainsi, il a été surprenant de constater que certaines combinaisons, comprenant par exemple de 15 à 50 pour cent environ de polyhexylméthacrylate, polyisobutylméthacrylate, polybutylméthacrylate, polyéthoxyéthylméthacrylate, polyacétate de vinyle comme premiers constituants polymérisé en présence du second, et de 85 à 50 pour cent de polycaprolactone ou d'un autre polymère comprenant au moins 80 pour cent d'unité structurale de type polyester aliphatique sont d'une rigidité et ont un pouvoir thermoadhésif suffisant pour assurer une résistance interlaminaire convenable dans un bandage ou une attelle à base de plaque pressée ou d'une étoffe imprégnée, alors que des mélanges mécaniques de 80 pour cent de polycaprolactone et 20 pour cent de copolymère ethylène-acétate de vinyle d'une teneur de 28 pour cent en acétate ne présentent pas de pouvoir thermoadhésif important.Ceci est d'autant plus surprenant que les copolymères d'éthylène-acétate de vinyle sont connus pour être des adhésifs "hot melt" alors que les polymères utilisés ne le sont pas.

De manière similaire, des combinaisons obtenues par synthèse de polyéthyldiglycol acrylate par exemple en proportion allant de 10 à 60 pour cent dans une quantité allant de 90 à 40% d'un polymère linéaire à base de polycaprolactone principalement donnent un produit thermoadhésif à prise rapide et dont la souplesse à température ambiante va en augmentant avec la proportion d'acrylate. Il a été en outre intéressant de remarquer que les combinaisons basées sur ces premiers constituants à base de polymères vinyliques ou (méth)acryliques peuvent être appliquées valablement sur un tricot à mailles ouvertes en utilisant 3 à 4 fois moins de solvant que la quantité nécessaire dans l'exécution du brevet des Etats-Unis d'Amérique 4,316,457.

On a trouvé également que certaines combinaisons spécifiquement intramoléculaires de type copolymères à blocs, linéaires, branchés ou greffés mais ne présentant pas une fraction de gel supérieure à 50 pour cent conviennent de la même manière si le copolymère comprend de manière distincte, des blocs ou greffons de type polyester aliphatique semi-cristallin d'une température de fusion comprise entre 35 et 80°C et d'un poids moléculaire individuel d'au moins environ 3000 et en proportion d'au moins 50 pour cent lesquels blocs définissent le second constituant, et des blocs, greffons ou branchements semi-cristallins ou non à température ambiante, mais ayant un caractère élastovisqueux à caoutchoutique au moins à la température de fusion du second constituant et définissant le premier constituant. D'un point de vue nomenclature, ce type de combinaison correspond plus spécifiquement à la notion d'homo-IPN.

Ainsi, notamment, on a trouvé que des préparations polyuréthannes à base de polyester diol d'une masse allant de 2500 à 5000, d'un triol dont les équivalents hydroxyles sont en proportion d'environ 3 à 15 pour cent du total des hydroxyles et d'un diisocyanate comme le toluylène diisocyanate ou l'hexaméthylène diisocyanate en proportion d'environ 92 à 98 pour cent par rapport à la stoéchiométrie donnent des résultats souhaités du point de vue fluidité contrôlée et adhésivité. Suivant le choix des proportions indiqués plus haut, ce matériau est, soit soluble et présente une distribution de poids moléculaires exceptionnellement large dont une fraction importante se situe au-delà de 50.000, soit constitué d'une fraction insoluble pouvant aller jusqu'à 50 pour cent et qui ne supprime pas les caractéristiques générales souhaitées.

Une autre combinaison intramoléculaire intéressante a été trouvée en préparant un polyuréthanne copolymère à blocs, en faisant réagir de 5 à 15 pour cent en poids des polyols caoutchoutiques comme le polytétraméthylèneglycol ou le polypropylèneoxyde diol ou polyol en présence de polyester diol de masse 3000 à 4500 et d'un diisocyanate en proportion environ stoéchiométrique, la rigidité la plus élevée à température ambiante étant obtenue avec un polytétraméthylèneglycol de masse au moins égale à 2900. En augmentant la proportion de polyol caoutchoutique jusqu'à 50% on obtient toujours un matériau thermomalléable et thermoadhésif mais qui devient de plus en plus souple quand la proportion augmente.

Dans un premier groupe de polymères permettant d'obtenir le premier constituant, la famille des polymères et copolymères vinyliques, d'ester acryliques ou méthacryliques s'est montrée particulièrement intéressante.

Le mode d'exécution préféré est donc de synthétiser l'un des deux constituants au moins en présence de l'autre, voire de les synthétiser tous les deux en même temps à partir d'un même mélange de tous les réactifs mais suivant deux mécanismes réactionnels différents. On obtient ainsi, par un procédé en masse, en solution ou en dispersion aqueuse, un alliage polymérique où les deux constituants sont dispersés l'un dans l'autre d'une manière quasi co-continue. Ce type de structure d'alliage répond en principe à la définition des réseaux polymères interpénétrés (IPN) ou à celle d'IPN thermoplastiques selon L.H. SPERLING dans "Interpenetrating Polymer Networks and Related Materials", Plenum press 1981, pages 3, 39 et 99. On pourrait concevoir que dans certains cas, le même résultat soit obtenu par une dissolution concomitante des deux polymères dans un même solvant ensuite évaporé, mais on imagine aisément que cette technique, d'ailleurs sujette à ségrégation moléculaire, serait trop onéreuse et dommageable pour l'environnement. Enfin, le mélange direct par malaxage, des deux constituants tels que précisés ne donne lieu qu'à une morphologie discrète d'une phase dispersée dans l'autre à moins que les deux constituants ne soient franchement miscibles.

Dans un deuxième groupe de polymères permettant de représenter le premier constituant , on a trouvé qu'il était possible d'obtenir les mêmes effets avec un copolymère comme par exemple de type polyuréthanne ou polyurée qui est de nature élastovisqueuse à caoutchoutique au moins à la température T2 et dans l'intervalle T2 à T4 voire en deça, selon que les caractéristiques de ce polyuréthanne gouvernent la température T4 ou que celle-ci est gouvernée par les caratéristiques du second constituant respectivement.

On obtient assez facilement ce constituant élastovisqueux à caoutchoutique au moins à la température T2, par réaction d'un mélange de diol, triol et diisocyanate notamment, mais on peut l'obtenir également au départ de réactifs difonctionnels uniquement si notamment, l'un deux est de nature intrinsèquement caoutchoutique comme le polytétraméthylèneglycol ou un polypropylèneoxyde. On peut évidemment employer en même temps un triol et un diol caoutchoutique.

Ce second type de premier constituant de nature polyuréthanne, peut par ailleurs être obtenu amorphe ou semi-cristallin à température ambiante mais dans ce dernier cas, sa température de ramollissement doit être inférieure à la température T2 visée, soit en tout cas inférieure à environ 95 à 100 °C et de préférence inférieure à 40°C. Il va de soi que la réaction uréthanne n'est pas indispensable pour former ce type de premier constituant dans la combinaison intramoléculaire et que toute autre liaison chimique peut convenir.

Dans un mode d'exécution préféré de l'invention, suivant cette deuxième famille de premiers constituants, notamment si on cherche à obtenir une rigidité à température ambiante aussi élevée que possible, on choisit un polyuréthanne contenant au moins 50 pour cent environ d'unités structurables cristallisables et ayant une température de fusion inférieure à T2, telles que a base d'ester aliphatique d'une masse moléculaire d'au moins 2000 environ et de préférence supérieure à 3000.

Tout comme pour la première famille de premiers constituants, on obtient, les meilleurs résultats, en synthétisant au moins un des deux constituants en présence de l'autre, voire les deux en même temps. La structure de l'alliage qui en résulte peut aussi être qualifiée d'IPN thermoplastique, voire d'homo-IPN thermoplastique.

Une combinaison particulièrement avantageuse est ainsi obtenue par polymérisation d'un mélange réactif contenant une même espèce cristallisable de masse moléculaire au moins égale à 2000 qui se partage entre les deux constituants formés. De manière préférée, cette espèce commune est un diol d'ester aliphatique comme le polycaprolactonediol ou un polyhexaméthylèneadipatediol par exemple, réagissant en présence d'un triol et d'un diisocyanate en défaut de quelques pour cent, voire 10 pour cent par rapport à la proportion stoéchiométrique, ou en présence de polytétraméthylèneglycol d'une masse moléculaire au moins égale à 2500 ou d'un polypropylèneoxydediol d'une même masse environ et d'un diisocyanate en proportion pratiquement stoéchiométrique. Certaines proportions de diol cristallisable et du triol, ou du diol cristallisable et du polytétraméthylèneglycol ou polypropylèneoxydediol donnent l'effet recherché d'un comportement élastovisqueux où le matériau a un bon pouvoir thermoadhésif tout en résistant à l'écoulement fluide.

Parmi ces combinaisons, on inclut également les combinaisons intramoléculaires du type copolymères à blocs où les séquences caoutchoutiques basées sur le polytétraméthylèneglycol notamment ont une masse moléculaire au moins égale à 2500.

Ces combinaisons apportent plusieurs avantages décisifs notamment par rapport aux polymères d'ester cyclique seuls et au polyuréthannes linéaires homopolymères du savoir-faire antérieur qui comme rappelé plus haut nécessite de manière optimale le type de substrat à brins volumineux décrit ainsi qu'un film intercalaire pour la préparation des bobines de bandage orthopédique.

Il faut aussi remarquer dès maintenant que la présente invention élargit considérablement le spectre des applications qui en découlent. Elle permet notamment d'obtenir un bandage thermoadhésif souple qui prouve son utilité pour le traitement d'une luxation telle que la tendinite. Elle permet de réaliser également des masses thermoadhésives de rigidité ou souplesse variables et d'un type essentiellement à prise rapide, des films ou feuilles thermoadhésifs et d'une épaisseur au choix permettant notamment de combler des cavités à la surface de corps granuleux tels que du béton ou de la brique tout en y adhérant, des plaques et feuilles thermoformables que l'on peut ensuite souder par endroits par chauffage localisé, etc.

Suivant les termes de la présente invention et en particulier lorsque le second constituant, au caractère thermoadhésif, est lui-même obtenu sous forme de polyuréthanne linéaire ou branché mais non réticulé, par réaction d'un diisocyanate avec un diol de type polyester aliphatique, en présence de la réaction donnant lieu au polyurétanne caoutchoutique basée en partie sur le même diol de polyester aliphatique, cette combinaison de polyuréthannes obtenus simultanément se distingue d'un homopolymère et en particulier d'un homopolymère linéaire, par le caractère en principe extractible du second constituant. Il peut s'agir d'une séparation de type sol-gel tout comme il peut s'agir d'une distinction par chromatographie ou simplement d'une distinction sur base du bilan matière des réactifs qui conduit nécessairement à la formation des deux espèces distinctes.

On peut d'ailleurs remarquer que le caractère hétérogène du matériau produisant l'effet recherché est obtenu en suivant notamment une procédure généralement évitée aussi bien dans la pratique des élastomères polyuréthannes que dans celles des polyuréthannes thermoplastiques à haut point de fusion, ce qui est nouveau. Dans la présente invention, on fait en effet réagir, notamment et par exemple, un mélange de polycaprolactonediol de masse 4300 avec une faible quantité de polycaprolactonetriol et un diisocyanate tel que l'hexaméthylènediisocyanate en quantité inférieure à la quantité stoéchiométrique d'environ 3 à 10 pour cent suivant la quantité de triol utilisée. On observe que le produit, élastovisqueux à chaud, conserve un bon pouvoir thermoadhésif alors que celui-ci est perdu si l'isocyanate intervient en proportion stoéchiométrique, et qu'en même temps dans ce dernier cas, le matériau est devenu élastomérique pur, ce qui correspond à la pratique des élastomères polyuréthannes.

On peut facilement juger du caractère élastovisqueux obtenu par la présente invention en observant, dans l'intervalle de température T2 à T4, un retrait partiel de la matière après allongement. Les polyesters linéaires ou les polyuréthannes linéaires qui en découlent manifestent quant à eux dans ces mêmes conditions une déformation fluide sans retrait, tandis que des polyuréthannes réticulés ont une rétraction de 100 pour cent tout en étant d'ailleurs beaucoup moins déformables et non adhésifs.

En résumé, les caractères du matériau obtenu avec la seconde famille du premier constituant sont tels qu'on y retrouve une contribution plastique et une contribution élastovisqueuse à caoutchoutique, la combinaison devenant elle-même élastovisqueuse.

Du point de vue procédé d'obtention de ce matériau, on trouve un avantage important par rapport au procédé décrit par le brevet des Etats-Unis d'Amérique 4,316,457, car il n'est plus nécessaire de polymériser en deux étapes tel que requis pour l'obtention du polyuréthanne linéaire homopolymère. Il a été trouvé au contraire qu'il était plus facile d'obtenir les propriétés utiles lorsque le caractère hétérodisperse du matériau est plus prononcé, ce qui est surprenant.

Comme autre conséquence avantageuse sur les caractéristiques physiques du produit, on a pu montrer que les températures T2 nécessaires avec ces combinaisons intramoléculaires sont de 5 à 10°C inférieures à celles nécessaires pour les polymères d'ester cyclique ou pour les polyuréthannes linéaires du savoir-faire antérieur alors que les températures T1 n'en sont que de 1 à 4°C inférieures ce qui est intéressant. En outre, le temps t4 est sensiblement allongé passant par exemple d'environ 1 à 2 minutes à environ 3 à 6 minutes ce qui devient un avantage important pour l'utilisateur peu habile ou chargé de réaliser un travail de grande dimension.

D'une manière générale, les applications des produits de la présente invention sont beaucoup plus larges que celles du savoir-faire antérieur. En effet, on peut obtenir des matières qui permettent de réaliser facilement, à la main, des structures telles que bandages circulaires, orthèses et prothèses externes et de maintien mais aussi des bandages thermoadhésifs souples qui n'étaient pas connus, de même que des éléments de structure décorative de grande dimension.

Les bandages orthopédiques thermoplastiques notamment peuvent être fabriqués directement en largeur utile de 5, 10 et 15 cm par exemple et être bordés de lisières uniformes et lisses. Les attelles et orthèses thermoplastiques obtenues par imprégnation de tissu peuvent être suivant la présente invention, dimensionnellment stables car celle-ci permet d'utiliser des tricots à brins de fibres continues, relativement serrés et résitant à l'allongement pendant l'étape d'imprégnation.

On peut moduler les températures caractéristiques beaucoup plus largement que par le savoir-faire antérieur et obtenir des souplesses et rigidités variables du matériau aussi bien pendant sa mise à forme qu'après refroidissement.

### Exemples

1. Dans un becher on a mélangé 100.5 grammes de polycaprolactone diol d'une masse moléculaire de 4280, 3.9 grammes de hexaméthylènediisocyanate, 34.6 grammes de méthacrylate de butyle, 0.03 ml de dibutyldilaurate d'étain et 0.4 grammes de trigonox 21. Le mélange a été coulé dans un ravier en aluminium et placé à l'étuve à 90°C.On a obtenu une masse qui après refroidissement est dure, rigide, et moulable. Le produit a été réchauffé à 80° C et moulé à la presse à plateaux en 3mm d'épaisseur. Traité à 60°C, la plaque se prête aisément au formage pendant un temps suffisant et ne coule pas sous son poids.
2. La préparation telle que décrite à l'exemple 1 a été diluée dans 70 parts de méthyléthylcétone pour 100 parts de résine. La solution a ét appliquée par trempage sur un tricot de fibres polyester/coton à mailles de 6mm et d'un titre de 151 grammes par mètre carré.Par traitement au four à 95% on obtient un bandage dont la largeur de 75mm permet d'obtenir directement une bobine par enroulement. Après trempage dans un bain d'eau à 60°C, on peut aisément dérouler la bobine et appliquer le bandage sur un membre. Le bandage se solidifie en 5 minutes.
3. Dans une préparation suivant l'exemple 1, on a uitilisé 43 grammes de méthacrylate de butyle au lieu de 34.6 grammes. Le matériau traité par DSC donne un taux de cristallinité de 67.91% alors que la même préparation sans le constituant méthacrylate a un taux de cristallinité de 68.48%. Cela signifie que dans la combinaison, le second constituant cristallise à 97% de son contenu, ce qui est surprenant. sur plaque moulée à la presse en 3mm d'épaisseur, on mesure une résistance efficace de 9.6 MPa et un allongement de la rupture de 28 pour cent. Le module d'élasticité à 22°C est de 512 MPa et les températures caractéristiques T1 et T2 sont respectivement 49°C et 60°C alors que pour l'HEXCELITE, ces températures sont respectivement 52 et 70°C.
4. Suivant les exemples 1 et 3, on a remplacé le butylméthacrylate successivement par le 2-éthylhexylméthacrylate (2EHMA),le 2-éthylhexylacrylate (2EHA), l'hexylméthacrylate (HMA) et l'isobutylméthacrylate (MAISOBU) en variant également les proportions en poids entre le premier et le second constituant comme indiqué dans le tableau ci-après. On trouve les valeurs indiquées pour différentes propriétés importantes qui montrent la diversité des applications possibles. On voit de nouveau que le premier constituant influence fortement le taux de cristallinité du second constituant, phénomène tout à fait inattendu et qui se répercute sur les propriétés physiques. On compare également aux propriétés du second constituant pris isolément.

| Proportion | Module Flexion (MPa) | Résis. mécan. (MPa) | Cristall. IPN (p.c.) | Cristall. ester (p.c.) | T2 (°C) |
|---|---|---|---|---|---|
| 2EHMA 30-70 | souple | 8,58 | 52,05 | 74,4 | 60 |
| 2EHA 30-70 | souple | 8,65 | 58,78 | 83.9 | 55 |
| 2EHA 40-60 | 63 | 6,88 | | | 55 |
| 2EHA 50-50 | 72 | 5,15 | | | 55 |
| HMA 30-70 | 104 | 8 | 51,79 | 74 | 60 |
| MAISOBOU 30-70 | 457 | 14,5 | 57,3 | 81,9 | 60 |
| MAISOBOU 40-60 | 479 | 16,5 | | | 60 |
| MAISOBOU 50-50 | 531 | 16,86 | | | 60 |
| néant 0-100 | 518 | 12,7 | 68,48 | 68,48 | 70 |

5. Suivant la procédure de l'exemple 1 on a remplacé le butylméthacrylate par de l'éthylglycolméthacrylate puis enfin par de l'éthyltriglycolacrylate. On obtient le même type de matériau qui devient de plus en plus souple en changeant de monomère dans l'ordre indiqué. Le matériau est un bon adhésif à la température de 55°C et redevient non collant après refroidissement à température ambiante.
6. On a préparé une formule adhésive sur le béton comme suit. Dans un bécher, on mélange 200g de polycaprolactone diol de masse 4280 à 65°C, 8,4 g de toluylène diisocyanate et 52,1 g respectivement de méthacrylate de butyle (MABU) et d'acrylate de butyle (ABU) ainsi que 1 ml de Trigonox 21 et 0,09 l de dibutyldilaurate d'étain. Le mélange est versé sur un non-tissé de polyester titrant 110g/mètre carré dans le cadre d'une presse à plateaux d'une épaisseur de 1,5 mm et chauffée à 65°C pendant 30 minutes puis à 100°C pendant 30 minutes. Après refroidissement, on obtient une feuille rigide et sèche que l'on applique sur une dalle en béton en chauffant à 80°C et en appliquant une pression de 1kg/cm carré ainsi qu'en ayant pris soin de laisser dépasser 15 cm de feuille sèche pour une essai de traction par pelage suivant la norme ASTM 903-49. Celui-ci donne une énergie de rupture supérieure à 7960 J/mètre carré pour le MABU et supérieur à 10.000 J/mètre carré pour l'ABU alors que le même second constituant tout seul donne une énergie de 6080 J/mètre carré. On a reproduit cette expérience en prenant des rapports en poids du premier au second constituant de 30/70, 40/60 et 50/50. Dans tous les cas, sauf pour 50 pour cent d'ABU, on a observé un mécanisme de rupture cohésive du béton, ce qui est remarquable. On a observé aussi que les courbes de modules d'élasticité en fonction de la température mesurées par analyse mécanique dynamique suivent une évolution tout à fait inattendue. D'une part, avec 20 pour cent d'ABU, le module est à tout moment supérieur à celui du second constituant tout seul, ce qui est surprenant vu que le polybutylacrylate est un caoutchouc d'une transition vitreuse à(-45°C). D'autre part, avec les diverses teneurs en MABU, le module est supérieur et à celui de second constituant seul jusqu'à une température de 33°C environ et d'autant plus que la teneur en MABU est élevée. Il y a ensuite croisement des courbes thermomécaniques et diminution des températures T1 et T2 lorsque la teneur en MABU augmente. Cela veut dire que par comparaison au second constituant seul, on obtient maintenant un matériau plus rigide à température ambiante mais qui se ramollit et peut être appliqué à une température plus basse, ce qui est tout à fait contraire aux résultats obtenus pour des mélanges mécaniques avec lesquels une diminution de température de transition s'accompagne toujours d'une diminution du module à température ambiante. On a obtenu l'évidence d'une morphologie IPN caractéristique en observant au microscope électronique, une coupe ultramince qui a pu être réalisée sur le composé à 50 pour cent de MABU. On voit clairement la topologie de la phase concentrée en MABU qui s'étire en chapelet au sein de la phase riche en ester.
7. Sur un tricot de 75 mm de large tel que décrit à l'exemple 2, on a appliqué par imprégnation, une solution dans le chlorure de méthylène dont la quantité a été ajustée pour obtenir une viscosité de 150 centipoises à 25°C, contenant 483,8 g de polycaprolactone diol de masse 4280, 3,4 g de polycaprolactonetriol d'un équivalent de 184, 19,7 g d'Hexaméthylènediisocyanate et 0,15 ml de dibutyldilaurate d'étain. Après traitement au four à 95°C pendant 40 minutes et refroidissement, on obtient un bandage qui, formé en roulette sans séparateur, peut être traité dans l'eau à 65°C puis être déroulé pour l'appliquer sur un membre sans qu'il y ait de transfert de matières sur les doigts de l'opérateur alors que le même bandage traité par les préparations donnant un polyuréthanne linéaire décrit dans l'art antérieur donne lieu à un transfert indésirable de résine sur les mains de l'opérateur. Après traitement à température T2 on observe que la résine déposée sur le substrat se déforme de manière élastovisqueuse et qu'il faut l'arracher avec une certaine force pour l'en dégager.
8. Suivant la technique décrite à l'exemple 2, on a imprégné en outre de la gaze en 10 cm de largeur qui après traitement au four a été laminée à la presse chauffante en 4 couches pour donner une épaisseur de 3 mm environ et en 3 couches pour donner une épaisseur de 2 mm environ. Ces laminats ont servi respectivement à une mesure de module en flexion à 22°C et à un suivi du module en torsion au cours du temps dans une ambiance à 22°C, après traitement à 70°C pendant 90 secondes, tous deux suivant la direction où la gaze elle-même n'a pas de résistance mécanique. La seconde mesure permet de définir les temps t3 et t4. On a également laminé le tricot imprégné en 4 couches par la technique de préparation d'attelles. Ces échantillons testés en flexion également présentent comme désiré, une porosité macroscopique allant de 0,3 à 0,45. On a obtenu les caractéristiques suivantes comparées à celles obtenues pour le même tricot et la même gaze imprégnées du polyuréthanne linéaire du savoir-faire antérieur.

| Premier constituant | Second constituant | Module flexion tricot (MPa) | Module flexion gaze (MPa) | t3 min | t4 min |
|---|---|---|---|---|---|
| néant | PU linéaire | 716 | 481 | 3,4 | 7,3 |
| 25 MABU | 75 | 640 | 446 | 3,1 | 6,0 |
| 20 MABU | 75 | 766 | 444 | 5,5 | 8,75 |
| 5 MAEE | | | | | |

On voit clairement qu'il devient possible de raccourcir ou d'allonger le temps de travail par rapport au savoirfaire antérieur sans modifier sensiblement la rigidité. En effet dans ce domaine, l'utilisateur est très sensible à des variations du temps de travail de l'ordre de la minute.

## Revendications

1. Combinaison moléculaire ou intramoléculaire de matières de type général IPN, dont l'un des constituants est synthétisé en présence de l'autre, associant un premier constituant polymère amorphe dans le domaine des températures supérieures à 40°C, ayant dans ladite combinaison un caractère élasto-visqueux à caoutchoutique à une température en dessous de 80°C, et un second constituant semi-cristallin comprenant 80% au moins, l'unité récurrente d'un ou plusieurs polyesters ayant une température moyenne de fusion cristalline comprise entre 35 et 80°C, caractérisée en ce que le premier constituant est, soit choisi parmi les polymères ou copolymères comprenant des espèces vinyliques, acryliques ou méthacryliques ou leurs mélanges et forme avec le second constituant un mélange de macromolécules, éventuellement greffés les uns aux autres, soit choisi parmi le groupe constitué par les polyéthers, polyesters, polyuréthanes ou leurs mélanges et forme avec le second constituant une combinaison intramoléculaire de segments macromères distincts.

2. Combinaison selon la revendication 1, où les deux constituants non réticulés sont partiellement ou totalement miscibles dans une morphologie de type semi-IPN thermoplastique.

3. Combinaison selon la revendication 1, où le premier constituant partiellement ou totalement réticulé et distinct du second, forme avec le second constituant non réticulé une dispersion partiellement ou totalement miscible dans une morphologie de type IPN thermoplastique.

4. Combinaison suivant la revendication 1, où le premier constituant partiellement ou totalement réticulé et distinct du second, forme avec le second constituant partiellement réticulé une dispersion partiellement ou totalement miscible dans une morphologie de type IPN élastomère lorsque le second constituant est à l'état fondu.

5. Procédé permettant d'obtenir la combinaison décrite à la revendication 2, en ce que le second constituant de type non réticulé et non réticulable est dissous dans le mélange de monomères monofonctionnels du premier constituant avec ou sans l'aide d'un solvant lesquels monomères sont amenés à polymériser par tout moyen de polymérisation dans la masse ou en solution ou après dépôt de la préparation au contact d'un substrat textile.

6. Procédé permettant d'obtenir la combinaison décrite à la revendiaction 3 en ce que le second constituant de type non réticulé et non réticulable est dissous dans le mélange de monomères du premier constituant comprenant un faible pourcentage et ne dépassant pas 5% de monomères plurifonnctionnels avec ou sans l'aide d'un solvant lesquels monomères sont amenés à polymériser dans la masse ou en solution ou après dépôt de la préparation au contact d'un substrat textile.

7. Procédé permettant d'obtenir la combinaison décrite à la revendication 4 en ce que le second constituant de type partiellement réticulable est dissous dans le mélange de monomères du premier constituant comprenant un faible pourcentage ne dépassant pas 5 pour cent de réactifs plurifonctionnels avec ou sans l'aide d'un solvant lesquels monomères sont amenés à polymériser par tout moyen de polymérisation et lequel second constituant est amené à réticuler partiellement.

8. Procédé permettant d'obtenir la combinaison décrite à la revendication 1, où le second constituant est synthétisé en présence du premier ou des moyens qui forment le premier avec ou sans l'aide d'un solvant, par réaction d'un mélange de polyisocyanates et de polyols lesquels polyols apportent en proportion d'au moins 80 pour cent de leur total une quantité de segments de type polyester semi-cristallin ayant une masse moléculaire individuelle moyenne d'au moins 2500 et ont une fonctionnalité moyenne n'excédant pas 2,5.

9. Procédé permettant d'obtenir la combinaison décrite à la revendication 4, où le second constituant est synthétisé en présence du premier ou des moyens qui forment le premier avec ou sans l'aide d'un solvant, par réaction d'un mélange de polyisocyanates et de polyols lesquels polyols apportent en proportion d'au moins 80 pour cent de leur total une quantité de segments de type polyester semi-cristallin ayant une masse moléculaire individuelle moyenne d'au moins 2500 et ont une fonctionnalité moyenne supérieure à 2,1.

10. Procédé suivant l'une quelconque des revendications 7 à 9 où les moyens de la combinaison sont appliqués sur un substrat textile et amenés à polymériser ensuite.

11. Matériau composite fait d'un substrat textile et d'une combinaison suivant l'une quelconque des revendications 1 à 4 en ce que la combinaison préparée selon un procédé où les moyens de la combinaison sont dispersés en phase aqueuse avec l'aide d'agents émulsifiants polymériques ou non et amenés à polymériser partiellement ou totalement en émulsion ladite composition est ensuite appliquée sur le substrat d'où l'eau est amenée à s'évaporer et la polymérisation à s'achever.

12. Combinaison thermoplastique intramoléculaire suivant la revendication 1, où les deux constituants se répartissent dans un polyuréthanne ou un polyurée branché et au plus partiellement réticulé caractérisé en ce qu'il comprend au moins 50 pour cent en poids d'unités structurales de type polyester semi-cristallin réparties en blocs ester-uréthanne lineaires d'une masse moyenne au moins égale à 15 000.

13. Combinaison thermoplastique intramoléculaire suivant la revendication 1, où les deux constituants se répartissent dans un polyuréthanne ou un polyurée copolymère à blocs caractérisé en ce qu'il comprend au moins 50 pour cent en poids d'unités structurales de type polyester semi-cristallin réparties en séquences esteruréthanne linéaires d'une masse moyenne au moins égale à 15 000.

14. Masse ou feuille thermoadhésive pour traitement à une température ne dépassant pas 100°C et à temps de redurcissement inférieur à 20 minutes comprenant la combinaison décrite à l'une quelconque des revendications 1 à 13.

15. Bandage orthopédique ou de maintien comprenant la combinaison décrite à l'une quelconque des revendications 1 à 13, formable et moulable par traitement à une température ne dépassant pas 80°C.

## Claims

1. A molecular or intramolecular combination of materials of the general IPN form, wherein one of the constituents is synthesised in the presence of the other, and comprising a first polymer constituent which is amorphous in the temperature domain above 40°C and having in said combination, an elastoviscous to rubbery behaviour at a temperature under 80°C and a second semi-crystalline constituent comprising 80 per cent at least of the recurring unit of one or several polyesters having an average crystalline melting temperature comprised between 35°C and 80°C, characterized in that the first constituent is either selected among the polymers or copolymers comprising vinyl acrylate, or methacrylate moieties or their mixtures and form with the second constituent a blend of macromolecules eventually grafted one to each other, or selected among the group of polyethers, polyesters, polyurethanes or their mixtures and form with the second constituent an intramolecular combination of distinct macromer segments.

2. A combination according to claim 1, wherein the two noncrosslinked constituents are partially or totally miscible in a morphology of a thermoplastic semi-IPN.

3. A combination according to claim 1, wherein the first constituent partially or totally crosslinked and distinct from the second forms with the second noncrosslinked constituent a dispersion partially or totally miscible in a morphology of a thermoplastic IPN.

4. A combination according to claim 1, wherein the first constituent partially or totally crosslinked and distinct from the second, forms with the second partially crosslinked constituent a partially or totally miscible dispersion in a morphology of an elastomer IPN when the second constituent is in the melt state.

5. A process for producing the combination described in claim 2, characterized in that the second noncrosslinked and noncrosslinkable constituent is dissolved in the mixture of monofunctional monomers of the first constituent with or without the aid of a solvent, said monomers are brought to polymerize by any means of polymerization in bulk, in solution or after such preparation has been placed in contact with a textile substrate.

6. A process for producing the combination described in claim 3, characterized in that the second noncrosslinked and noncrosslinkable constituent is dissolved in the mixture of monomers of the first constituent comprising a small amount not exceeding 5 percent of multifunctional monomers with or without the aid of a solvent, said monomers are brought to polymerize in bulk or in solution or after the preparation has been placed in contact with a textile substrate.

7. A process for producing the combination described in claim 4, characterized in that the second partially crosslinkable constituent is dissolved in the mixture of monomers of the first constituent comprising a small amount not exceeding 5 percent of multifunctional moieties with or without the aid of a solvent said monomers being brought to polymerize by any means of polymerization and said second constituent being caused to crosslink partially.

8. A process for producing the combination described in claim 1, wherein the second constituent is synthesised in the presence of the first one or of the means to obtain the first one, with or without the aid of a solvent, by reaction a of mixture of polyisocyanates and polyols, said polyols containing an amount of at least 80 per cent of semi-crystalline polyester macromer segments having an individual average molecular weight of at least 2500 and said polyols having an average functionality not exceeding 2.5.

9. A process for producing the combination described in claim 1, wherein the second constituent is synthesised in the presence of the first one or of the means to obtain the first one, with or without the aid of a solvent, by reaction of a mixture of polyisocyanates and polyols, said polyols containing an amount of at least 80 per cent of semi-crystalline polyester macromer segments having an individual average molecular weight of at least 2500 and said polyols having an average functionality greater than 2,1.

10. A process according to one of claims 7 to 9, wherein the means of the combination are applied onto a textile substrate and brought to polymerize afterwards.

11. A composite material consisting of a textile substrate and a combination according to one of claims 1 to 4, characterized in that the combination prepared according to a process wherein the means of the combination are dispersed in an aqueous medium with the aid of polymeric or nonpolymeric emulsifying agents and are brought to polymerize partially or totally in emulsion, said composition is further applied onto the substrate from which the water is brought to evaporate and on which the polymerization is carried on to completion.

12. An intramolecular thermoplastic combination according to claim 1, wherein the two constituents are distributed in a branched polyurethane or polyurea at most partially crosslinked characterized in that it comprises at least 50 per cent by weight of semi-crystalline polyester recurring units distributed in linear ester-urethane blocks having an average molecular weight of at least 15000.

13. An intramolecular thermoplastic combination according to claim 1, wherein the two constituents are distributed in a urethane or urea block copolymer characterized in that it comprises at least 50 per cent by weight of semi-crystalline polyester recurring units distributed in linear ester-urethane blocks having an average molecular weight of at least 15000.

14. A bulk material or sheet, thermoadhesive by treatment at a temperature under 100°C that further sets under cooling in a time less than 20 minutes and comprising the combination described in one of claims 1 to 13.

15. An orthopaedic or supporting bandage comprising the combination described in one of claims 1 to 13, formable and mouldable after treatment at a temperature not exceeding 80°C.

## Patentansprüche

1. Molekulare oder intramolekulare Stoffverbindung von dem allgemeinen Typ IPN, bei der einer der Bestandteile in Gegenwart des anderen synthetisiert wird, und bei der ein erster polymerer Bestandteil, der in dem Temperaturbereich über 40°C amorph ist, und bei einer Temperatur unter 80°C in der Stoffverbindung ein elastoviskoses bis gummiartiges Verhalten zeigt, und ein zweiter, halbkristalliner Bestandteil, der zu mindestens 80% die sich wiederholende Einheit aus einem oder mehreren Polyestern mit einer mittleren kristallinen Schmelztemperatur zwischen 35 und 80°C aufweist, miteinander kombiniert sind, dadurch gekennzeichnet, daß der erste Bestandteil entweder unter den Polymeren oder Copolymeren, die Vinyl-, Acryl- oder Methacrylarten aufweisen, oder ihren Gemischen gewählt wird, und mit dem zweiten Bestandteil ein Gemisch von Makromolekülen bildet, die eventuell aufeinander gepfropft sind, oder innerhalb der aus Polyäthern, Polyestern, Polyurethanen oder ihren Gemischen bestehenden Gruppe gewählt wird, und mit dem zweiten Bestandteil eine intramolekulare Stoffverbindung mit verschiedenen makromeren Segmenten bildet.

2. Stoffverbindung gemäß Anspruch 1, wobei die zwei nicht vernetzten Bestandteile bei einer Morphologie vom Typ thermoplastisches Halb-IPN teilweise oder vollständig mischbar sind.

3. Stoffverbindung gemäß Anspruch 1, wobei der teilweise oder vollständig vernetzte, erste Bestandteil, der verschieden von dem zweiten ist, mit dem nicht vernetzten, zweiten Bestandteil eine Dispersion bildet, die bei einer Morphologie vom Typ thermoplastisches IPN teilweise oder vollständig mischbar ist.

4. Stoffverbindung gemäß Anspruch 1, wobei der teilweise oder vollständig vernetzte, erste Bestandteil, der verschieden von dem zweiten ist, mit dem teilweise vernetzten, zweiten Bestandteil eine Dispersion bildet, die bei einer Morphologie vom Typ elastomeres IPN teilweise oder vollständig mischbar ist, wenn der zweite Bestandteil im geschmolzenen Zustand ist.

5. Verfahren, das ermöglicht, die in dem Anspruch 2 beschriebene Stoffverbindung zu erhalten, wozu der zweite Bestandteil vom nicht vernetzten und nicht vernetzbaren Typ in dem Gemisch von monofunktionalen Monomeren des ersten Bestandteils mit oder ohne Hilfe eines Lösungsmittels aufgelöst wird, wobei diese Monomere durch jedes Mittel zur Polymerisation in der Masse oder in Lösung oder nach Ablagerung der zubereiteten Mischung beim Kontakt mit einem Textilsubstrat zur Polymerisation gebracht werden.

6. Verfahren, das ermöglicht, die in dem Anspruch 3 beschriebene Stoffverbindung zu erhalten, wozu der zweite Bestandteil vom nicht vernetzten und nicht vernetzbaren Typ in dem Gemisch von Monomeren des ersten Bestandteils, das einen geringen Prozentsatz, der 5% nicht übersteigt, von plurifunktionalen Monomeren aufweist, mit oder ohne Hilfe eines Lösungsmittels aufgelöst wird, wobei diese Monomere in der Masse oder in Lösung oder nach Ablagerung der zubereiteten Mischung beim Kontakt mit einem Textilsubstrat zur Polymerisation gebracht werden.

7. Verfahren, das ermöglicht, die in Anspruch 4 beschriebene Stoffverbindung zu erhalten, wozu der zweite Bestandteil vom teilweise vernetzbaren Typ in dem Gemisch von Monomeren des ersten Bestandteils, das einen geringen Prozentsatz, der 5% nicht übersteigt, von plurifunktionalen Reagenzien aufweist, mit oder ohne Hilfe eines Lösungsmittels aufgelöst wird, wobei diese Monomere durch jedes Polymerisationsmittel zur Polymerisation gebracht werden, und der zweite Bestandteil dazu gebracht wird, teilweise zu vernetzen.

8. Verfahren, das ermöglicht, die in dem Anspruch 1 beschriebene Stoffverbindung zu erhalten, wobei der zweite Bestandteil in Gegenwart des ersten, oder der Mittel, die den ersten bilden, mit oder ohne Hilfe eines Lösungsmittels durch Reaktion eines Gemischs von Polyisocyanaten und Polyolen synthetisiert wird, wobei diese Polyole bei einem Anteil von mindestens 80 Prozent ihrer Gesamtmenge eine Menge von Segmenten vom Typ halbkristalliner Polyester mit einer mittleren, individuellen molaren Masse von mindestens 2500 einbringen, und eine mittlere Funktionalität haben, die 2,5 nicht übersteigt.

9. Verfahren, das ermöglicht, die in dem Anspruch 4 beschriebene Stoffverbindung zu erhalten, wobei der zweite Bestandteil in Gegenwart des ersten, oder der Mittel, die den ersten bilden, mit oder ohne Hilfe eines Lösungsmittels durch Reaktion eines Gemischs von Polyisocyanaten und Polyolen synthetisiert wird, wobei diese Polyole bei einem Anteil von mindestens 80 Prozent ihrer Gesamtmenge eine Menge von Segmenten vom Typ halbkristalliner Polyester mit einer mittleren, individuellen molaren Masse von mindestens 2500 einbringen, und eine mittlere Funktionalität haben, die größer als 2,1 ist.

10. Verfahren gemäß irgendeinem der Ansprüche 7 bis 9, wobei die Mittel der Stoffverbindung auf ein Textilsubstrat aufgebracht werden und danach zur Polymerisation gebracht werden.

11. Verbundstoff aus einem Textilsubstrat und einer Stoffverbindung gemäß irgendeinem der Ansprüche 1 bis 4, wobei die Stoffverbindung gemäß einem Verfahren hergestellt wird, bei dem die Mittel der Stoffverbindung in wässeriger Phase mit Hilfe von polymeren oder nichtpolymeren Emulgierungsmitteln dispergiert werden, und dazu gebracht werden, in der Emulsion teilweise oder vollständig zu polymerisieren, wobei die Zusammensetzung danach auf das Substrat aufgebracht wird, wo das Wasser verdampft wird, und die Polymerisation zu Ende geführt wird.

12. Intramolekulare, thermoplastische Stoffverbindung gemäß Anspruch 1, wobei die zwei Bestandteile sich ein verweigtes oder höchstens teilweise vernetztes Polyurethan- oder Polyharnstoff-Copolymer teilen, dadurch gekennzeichnet, daß es mindestens 50 Gewichtsprozent strukturelle Einheiten vom Typ halbkristalliner Polyester enthält, die in lineare Ester-Urethan-Blöcke mit einer mittleren Masse von mindestens 15 000 aufgeteilt sind.

13. Intramolekulare, thermoplastische Stoffverbindung gemäß Anspruch 1, wobei die zwei Bestandteile sich ein Polyurethan- oder Polyharnstoff-Copolymer mit Blöcken teilen, dadurch gekennzeichnet, daß es mindestens 50 Gewichtsprozent strukturelle Einheiten vom Typ halbkristalliner Polyester enthält, die in lineare Ester-Urethan-Sequenzen mit einer mittleren Masse von mindestens 15 000 aufgeteilt sind.

14. Thermoklebende Masse oder Folie zur Verarbeitung bei einer Temperatur von nicht mehr als 100°C, und mit einer Wiedererhärtungszeit von weniger als 20 Minuten, die die in irgendeinem der Ansprüche 1 bis 13 beschriebene Stoffverbindung enthält.

15. Orthopädische Bandage oder Haltebandage, die die in irgendeinem der Ansprüche 1 bis 13 beschriebene Stoffverbindung aufweist, die durch Verarbeitung bei einer Temperatur von nicht mehr als 80°C formbar und preßformbar ist.
